# FASCICULE DE BREVET EUROPEEN

(11) **EP 2 588 077 B1**
(45) Date de publication et mention de la délivrance du brevet: **13.06.2018**
(21) Numéro de dépôt: 11741478.9
(22) Date de dépôt: 29.06.2011
(51) Int. Cl.: A61K 8/88, A61Q 19/00

(54) **COMPOSITION COSMETIQUE COMPRENANT DU COPA**
KOSMETISCHE ZUBEREITUNG ENTHALTEND COPA
COSMETIC COMPOSITION CONTAINING COPA

(30) Priorité: 01.07.2010 FR 1002790
(43) Date de publication de la demande: 08.05.2013
(73) Titulaire: Arkema France, 92700 Colombes (FR)
(72) Inventeur: PINEAU, Quentin, F-27000 Evreux (FR)
(74) Mandataire: Hérard, Elise
(86) Numéro de dépôt international: PCT/FR2011/051510
(87) Numéro de publication internationale: WO 2012/001299

(56) Documents cités:
- EP-A1- 1 661 546
- WO-A2-2008/145889
- FR-A1- 2 939 802
- US-A- 4 097 589

## Description

### Domaine de l'invention

La présente invention a pour objet une composition cosmétique comprenant un polymère thermoplastique, en particulier un copolyamide tel que défini dans la revendication 1 (ci-après COPA).

La présente invention se rapporte en particulier à l'utilisation de ce COPA tel que défini dans la revendication 17, dans les produits cosmétiques, pharmaceutiques ou de parfumerie, et aux compostions cosmétiques, pharmaceutiques, ou de parfumerie comprenant au moins un COPA.

### Technique antérieure

Les polymères thermoplastiques destinés à une utilisation cosmétique ou médicale se présentent généralement sous forme de poudre. Par conséquent, la formulation avec ces poudres nécessite généralement des étapes intermédiaires de broyage, voire de tamisage, sous forme de poudre fine de D50 généralement inférieure à 10 ou 20 µm, et de dispersion préalable de cette poudre dans un liquide pour que la texture du produit final incorporant la poudre présente une parfaite homogénéité et un aspect uniforme. De plus, la grande volatilité des poudres fines fait que leur utilisation en formulation nécessite beaucoup de précautions. Il est notamment difficile pour les formulateurs de quantifier exactement et de façon reproductible la teneur en poudre des formules.

Parmi les polymères thermoplastiques, les copolyamides (ci-après COPA) sont connus comme adhésifs du type "hot melt adhesives" (ou HMA), c'est-à-dire qu'on les dépose à l'état fondu sur les surfaces à coller, l'adhésion étant ensuite obtenue par refroidissement, par le retour à l'état solide des copolyamides. Le point de fusion de ces COPA est compris dans la gamme de 80 à 190°C, de préférence de 100 à 130°C. La composition en monomères et le ratio en poids entre les monomères déterminent notamment les propriétés d'adhésion sur différents supports et la résistance chimique de ces COPA. Leurs propriétés sont déjà exploitées dans le textile, dans la fabrication de films, filaments, voiles ou grilles, de vernis, de peintures, d'encres et le revêtement de matériaux, notamment par des poudres fines de COPA. En revanche, ces propriétés des COPAs n'ont jamais été exploitées dans le domaine cosmétique, où les défis lancés aux formulateurs en terme de textures et de toucher sont de plus en plus exigeants et variés.

La présente invention a donc pour but de fournir des compositions comprenant du COPA, prêtes à l'emploi, qui facilitent la mise en oeuvre du COPA par les formulateurs, en étant directement utilisables (par simple incorporation) dans des formulations cosmétiques, par exemple pour leur conférer une texture particulière. Ainsi, le formulateur n'a plus besoin d'adapter au préalable la forme des COPAs, vendus dans le commerce sous forme de poudre ou de granulés. De plus, les teneurs en COPA dans les formulations sont facilement quantifiables et reproductibles.

La présente invention a notamment pour but de fournir un procédé simple (comprenant le moins d'étapes possibles) de fabrication de telles compositions de COPA prêtes à l'emploi.

De manière surprenante, la Demanderesse a également montré que l'utilisation de copolyamides permettait de fabriquer de telles compositions cosmétiques aux textures et propriétés innovantes.

### Description détaillée de l'invention

La présente invention a donc pour objet une composition tel que définie dans la revendication 1.

### COPA:

Les « copolyamide » abrégés « COPA » comprennent au moins deux motifs répétitifs distincts, ces motifs distincts étant formés à partir d'au moins deux monomères différents. Les copolyamides sont donc préparés à partir de deux ou plusieurs comonomères choisis parmi un aminoacide, un lactame et/ou un diacide carboxylique avec une diamine.

Le terme « monomère » dans la présente description des copolyamides doit être pris au sens d' « unité répétitive » ou « motif répétitif ». En effet, le cas où une unité répétitive du PA est constituée de l'association d'un diacide avec une diamine est particulier. On considère que c'est l'association d'une diamine et d'un diacide, c'est-à-dire le couple diamine.diacide (en quantité équimolaire), qui correspond au monomère. Ceci s'explique par le fait qu'individuellement, le diacide ou la diamine n'est qu'une unité structurale, qui ne suffit pas à elle seule à polymériser.

Par copolyamide (abrégé CoPA), on entend les produits de polymérisation d'au moins deux monomères différents choisis parmi :
- les monomères de type aminoacides ou acides aminocarboxyliques, et de préférence les acides alpha,oméga-aminocarboxyliques;
- les monomères de type lactames ayant de 3 à 18 atomes de carbone sur le cycle principal et pouvant être substitués ;
- les monomères de type « diamine.diacide » issus de la réaction entre une diamine aliphatique ayant de 4 et 36 atomes de carbone et un diacide carboxylique ayant de 4 et 36 atomes de carbone; et
- leurs mélanges, avec des monomères à nombre de carbone différent dans le cas de mélanges entre un monomère de type aminoacide et un monomère de type lactame.

### Monomères de type aminoacides :

Avantageusement, le COPA utilisé dans la composition selon l'invention est obtenu au moins partiellement à partir de matières premières bio-ressourcées.
Par matières premières d'origine renouvelable ou matières premières bio-ressourcées, on entend des matériaux qui comprennent du carbone bio-ressourcé ou carbone d'origine renouvelable. En effet, à la différence des matériaux issus de matières fossiles, les matériaux composés de matières premières renouvelables contiennent du ¹⁴C. La « teneur en carbone d'origine renouvelable » ou « teneur en carbone bio-ressourcé » est déterminée en application des normes ASTM D 6866 (ASTM D 6866-06) et ASTM D 7026 (ASTM D 7026-04). A titre d'exemple, les COPA à base de polyamide 11 proviennent au moins en partie de matières premières bio-ressourcées et présentent une teneur en carbone bio-ressourcé d'au moins 1%, ce qui correspond à un ratio isotopique de ¹²C/¹⁴C d'au moins 1,2 x 10⁻¹⁴. De préférence, les COPA selon l'invention comprennent au moins 50% en masse de carbone bio-ressourcé sur la masse totale de carbone, ce qui correspond à un ratio isotopique ¹²C/¹⁴C d'au moins 0,6.10⁻¹². Cette teneur est avantageusement plus élevée, notamment jusqu'à 100%, qui correspond à un ratio isotopique ¹²C/¹⁴C de 1,2 x 10⁻¹², dans le cas de COPAs issus en totalité de matières premières d'orgine renouvelable.

A titre d'exemples d'aminoacides d'origine renouvelable, on peut citer: l'acide 11-aminoundecanoïque produit à partir d'huile de ricin par exemple, l'acide 12-aminododecanoïque produits à partir d'huile de ricin par exemple, l'acide 10-aminodécanoïque produit à partir de l'acide decylénique obtenu par métathèse de l'acide oléïque par exemple, l'acide 9-aminononanoïque produit à partir de l'acide oléïque par exemple.

A titre d'exemples de diacides d'origine renouvelable, on peut citer, en fonction du nombre x de carbones de la molécule (Cx) :
- C4 : l'acide succinique à partir du glucose par exemple ;
- C6 : l'acide adipique à partir du glucose par exemple ;
- C7 : l'acide heptanedioïque à partir d'huile de ricin ;
- C9 : l'acide azélaïque à partir de l'acide oléïque (ozonolyse) par exemple ;
- C10 : l'acide sébacique à partir de l'huile de ricin par exemple ;
- C11 : l'acide undécanedioïque à partir d'huile de ricin ;
- C12 : l'acide dodécanedioïque à partir de biofermentation de l'acide dodecanoïque = acide laurique (huile riche : huile de palmiste et noix de coco) par exemple ;
- C13 : acide brassylique à partir de l'acide erucique (ozonolyse) que l'on trouve dans le colza par exemple ;
- C14 : acide tetradécanedioïque par biofermentation de l'acide myristique (huile riche : huile de palmiste et noix de coco) par exemple ;
- C16 : acide hexadécanedioïque par biofermentation de l'acide palmitique (huile de palme principalement) par exemple ;
- C18 : acide octadécanedioïque obtenu par biofermentation de l'acide stéarique (un peu dans toutes les huiles végétales mais majoritaire dans les graisses animales) par exemple ;
- C20 : acide eicosanedioïque obtenu par biofermentation de l'acide arachidique (majoritaire dans l'huile de colza) par exemple ;
- C22 : acide docosanedioïque obtenu par métathèse de l'acide undécylénique (huile de ricin) par exemple
- C36 : dimère d'acide gras issu des sous-produits des résineux transfomés par les procédé Kraft.

A titre d'exemples de diamines d'origine renouvelable, on peut citer, en fonction du nombre x de carbones de la molécule (Cx) :
- C4 : butanediamine obtenu par amination de l'acide succinique ;
- C5 : pentaméthylene diamine (à partir de lysine) ;
et ainsi de suite pour les diamines obtenues par amination des diacides d'origine renouvelable vus précédemment.
Par copolyamide d'origine totalement renouvelable, on entend les copolyamides résultant de la polymérisation de divers monomères (renouvelables, non renouvelables ou mixtes) tels que ceux cités ci-dessus. C'est le cas par exemple du CoPA 6.6/10.10 dans lequel le monomère « 6.6 » est d'origine non renouvelable tandis que le monomère « 10.10 » est d'origine renouvelable.
A titre d'exemples de copolyamides, on peut notamment citer ceux commercialisés sous le nom « Platamid® » et Platamid Rnew® par ARKEMA.

Par milieu acceptable en cosmétique, parfumerie ou pharmacie, on entend au sens de l'invention tout produit qui ne provoque pas d'irritation ni de réaction sur la peau, les fibres kératiniques (cils, cheveux) ou les ongles.

Avantageusement, ledit milieu, comprenant au moins 50% d'eau, au moins un composant choisi parmi les alcools, les solutions alcooliques, les composés lipidiques, les composés glucidiques, les hydrocarbures, les polymères synthétiques, polymères naturels, et/ou les extraits végétaux.

Avantageusement, la composition selon l'invention a au moins l'une des formes suivantes : dispersion, solution, émulsion, microémulsion, nanoémulsion, émulsion sèche, suspension, aérosol, gel, gel compact, gomme, gomme plastique, pâte, mousse, crème, poudre, poudre libre, poudre compacte, poudre expansée, beurre, film, film élastique, et leurs mélanges. Ledit milieu comprend une phase aqueuse comprenant au moins 50% d'eau. L'eau peut être de l'eau adoucie, de l'eau déminéralisée et/ou de l'eau stérilisée, selon son degré de purifications, ou de l'eau thermale. La phase aqueuse peut en outre comprendre des alcools dont le nombre de carbones de la chaîne carbonée ne dépasse pas 6, qui sont hydrosolubles, tels que l'éthanol, l'isopropanol. Des solutions alcooliques obtenues par simple mélange de ces alcools avec l'eau sont également utilisables dans la phase aqueuse ; de même que les glycols, comme l'éthylène glycol, propylène glycol ; les polyols, comme le glycérol ou glycérine, le sorbitol, le sirop de sorbitol.

Les polyoxyéthylènes glycols (PEG) peuvent également être utilisés comme solvants dans ladite phase aqueuse. Les polymères carboxyvinyliques (carbomères ou Carbopol), les polymères cyanoacryliques ; les composés glucidiques, tels que polysaccharides ou polyosides extraits d'algues (alginates, carragénates), du bois (cellulose et ses dérivés), de la sève des arbres (gomme arabique, gomme adragante), de graines ou de pépins (pectine, gommes de guar, de caroube, amidon), de feuilles (gel d'aloès) ; les glycoprotéines ou protéoglycanes ; les esters et éthers glucidiques peuvent également entrer dans la composition de la phase aqueuse, notamment comme épaississants ou gélifiants de la phase aqueuse.

Les conservateurs, émulsionnants hydrophiles, colorants, humectants, gélifiants, actifs hydrophiles et tout autre agent cosmétique hydrophile peuvent entrer dans la composition de ladite phase acqeuse ou hydrophile.

Avantageusement, ledit milieu comprend une phase huileuse comprenant au moins 50% de solvant organique choisi parmi les esters gras, les alcools gras, les acides gras, et leurs mélanges.

Les alcools gras au sens de l'invention sont des alcools dont la chaîne carbonée comprend au moins 7 carbones, de préférence de 7 à 10 carbones, de sorte qu'ils sont liquides à température ambiante. Ils sont insolubles dans l'eau mais la présence de l'hydroxyle leur confère une faible affinité pour l'eau. On peut notamment citer l'alcool benzylique qui joue le rôle de solvant et de conservateur.

Les acides gras au sens de l'invention sont des acides organiques qui se trouvent dans les lipides. Leur chaîne carbonée est plus ou moins longue (de C4 à C30) et ils peuvent être saturés ou insaturés. Les acides gras saturés sont solides à la température ambiante (25°C), sauf les acides en C4 et C6. Les acides gras insaturés sont liquides. A titre d'exemple, on peut citer l'acide laurique, l'acide stéarique, l'acide oléique.

Les esters gras résultent soit de la combinaison d'un acide gras avec un alcool à chaîne courte (par exemple le palmitate ou myristate d'isopropyle qui forment des esters gras liquides), soit de la combinaison d'un acide gras avec un alcool gras (par exemple l'isostéarate d'isostéaryle), soit de la combinaison d'un acide à chaîne courte avec un alcool gras à chaîne plus ou moins longue (par exemple acide benzoïque avec un alcool gras en C13-C15 formant des benzoates d'alcools gras).

D'autres composés lipidiques peuvent se trouver dans ladite phase huileuse, tels que les huiles végétales comme l'huile de jojoba, l'huile de ricin, l'huile d'arachide, l'huile de tournesol, l'huile de bourrache, l'huile de coco ; les huiles animales ; ces huiles pouvant être modifiées chimiquement ou non ; les huiles synthétiques comme les mono-, di- et tri-glycérides synthétiques, par exemple les triglycérides capryliques capriques ; les insaponifiables, notamment d'avocat, de soja, de maïs, de karité ; les beurres, notamment de karité, de coprah, de cacao ; les cires de point de fusion supérieur à 50°C d'origine végétale (cire de carnauba, de candelilla) ou animale (cire d'abeille, propolis) ; les phospholipides, notamment lécithine de soja.

Ladite phase huileuse peut encore inclure des hydrocarbures minéraux : huiles minérales, vaselines, paraffines, isoparaffines ; des silicones comme les huiles silicones telles que diméthicone, phénylméthicone, les silicones volatils telles que la cyclométhicone ou les silicones émulsionnants.

La phase huileuse peut également contenir des silices, des silicates, notamment silicate d'aluminium et/ou de magnésium, argile, kaolin, montmorillonite, bentone, bentonite, hectorite qui épaississent la phase huileuse.

Des antioxydants, pigments, charges comme le talc, nylon, silice, des actifs lipophiles et tout autre agent cosmétique lipophile peuvent également entrer dans la composition de ladite phase huileuse.

De manière avantageuse, ledit milieu comprend en outre de 0,1 à 30% en poids de tensioactif sur le poids total de COPA.

Par tensioactif au sens de l'invention, on entend :
- les tensioactifs d'origine synthétique qui se divisent eux-mêmes en deux groupes, tensioactifs ioniques (anioniques comme le lauryl sulfate de sodium ou les sels d'acide gras, cationiques comme le chlorure de stéaryl ammonium, ou amphotères comme les dérivés de bétaïne) et les tensioactifs non ioniques de HLB compris dans la gamme de 0 à 20, par exemple les esters de sorbitan (Span, Tween) et les alcools gras polyoxyéthylénés ; et
- les tensioactifs d'origine naturelle, comme le cholestérol, la lécithine, la saponine, et les protéines telles que la caséine.

De préférence, le tensioactif selon l'invention est choisi parmi les tensioactifs anioniques et les tensioactifs non ioniques. Le tensioactif est avantageusement choisi parmi les sels de sulfate d'éther d'alkyle et de polyoxyalkylène, les sels de dialkylsulfosuccinate, les sels d'acide gras et les copolyamides d'oxyde d'éthylène/oxyde de propylène, et leurs mélanges.

Avantageusement, la composition selon l'invention comprend en outre un épaississant ou gélifiant lipophile ou hydrophile tel que ceux décrits précédemment.
Selon un mode de réalisation préféré de l'invention, la composition forme une dispersion de COPA dissous dans une phase huileuse dispersée dans une phase aqueuse en présence d'un tensioactif.
Selon un autre mode de réalisation avantageux de l'invention, la composition forme une dispersion de COPA dissous dans une phase aqueuse dispersée dans une phase huileuse en présence d'un tensioactif.

La présente invention a également pour objet un procédé d'incorporation d'un copolyamide dans un milieu cosmétique, de parfumerie et/ou pharmaceutique, ledit procédé comprenant au moins une étape choisie parmi : mélanger, disperser, homogénéiser, homogénéiser par haute pression, mettre en solution, diluer, dissoudre, gélifier, épaissir, émulsionner, affiner, empâter, traiter thermiquement, sécher, lyophiliser, cuire, extruder, broyer, granuler, atomiser, filtrer, et les mélanges successifs ou simultanés de plusieurs de ces étapes.

Avantageusement, ledit procédé comprend les étapes de :
- ajout direct ou progressif, de préférence sous agitation comprise dans la gamme de 100 à 20 000 tr/min, de copolyamide (COPA) dans un milieu acceptable en cosmétique, en parfumerie, et/ou en pharmacie, le ratio en poids COPA/milieu étant compris dans la gamme de 40/60 à 1/99, les bornes de la gamme étant incluses; et
- chauffage dudit mélange à une température comprise dans la gamme de 40 à 190°C, de préférence dans la gamme de 60 à 180°C, de préférence de 70 à 130°C, de préférence sous une pression comprise dans la gamme 1 à 100 bar, pendant une durée comprise dans la gamme de 5 à 120 minutes, de préférence de 5 à 60 minutes, de préférence de 10 à 30 minutes ;
les étapes d'ajout et de chauffage étant soit simultanées, soit successives prises dans cet ordre ou dans l'ordre inverse. Le procédé selon l'invention comprend l'ajout de 0,1 à 30% de copolyamide (COPA) dans 70 à 99,9% d'un milieu acceptable en cosmétique et/ou en parfumerie, le mélange obtenu représentant 100%.

De préférence, le COPA utilisé dans le procédé de l'invention est sous forme de poudre de D50 compris dans la gamme de 1 à 150 µm, de préférence dans la gamme de 1 à 100 µm, de préférence de 1 à 50 µm, de préférence de 1 à 30 µm.

Au sens de l'invention, le D50 correspondant à la taille moyenne en volume, c'est à dire la valeur de la taille de particule qui divise la population de particules examinée exactement en deux. Le D50 est mesuré selon la norme ISO 9276 - parties 1 à 6 : « Représentation de données obtenues par analyse granulométrique ». Dans le procédé de l'invention, le milieu acceptable en cosmétique, en parfumerie et/ou en pharmacie comprend une phase aqueuse (ou un solvent polaire, de préférence protique) comprenant au moins 50% d'eau sur le poids de phase aqueuse et une phase huileuse comprenant au moins 50% de solvant organique sur le poids de phase huileuse, ledit solvant organique étant choisi parmi les esters gras, les alcools gras, les acides gras et leurs mélanges.

Selon un mode de réalisation avantageux, le procédé de l'invention comprend les étapes suivantes :
- dissoudre le COPA dans la phase huileuse ;
- émulsionner la solution de COPA obtenue dans la phase aqueuse contenant au moins un tensioactif, choisi de préférence parmi les tensioactifs anioniques et les tensioactifs non ioniques.
Selon un mode de réalisation préféré, le procédé de l'invention comprend un étape consistant à disperser du COPA de température de fusion Tf dans la phase aqueuse contenant au moins un composé basique et/ou un tensioactif choisi parmi les tensioactifs anioniques et les tensioactifs non ioniques, à une température supérieure ou égale à Tf, et sous agitation à taux de cisaillement suffisant pour que le D50 des gouttes de COPA émulsionnées soit dans la gamme de 0,1 à 50 µm, de préférence dans la gamme de 0,1 à 20 µm, dans la gamme de 0,1 à 10 µm.
De préférence, le COPA est incorporé selon le procédé de l'invention, sous agitation comprise dans la gamme de 100 à 15 000 tours par minute, de préférence de 1000 tr/min à 12 000 tr/min, parfois à plus de 8 000 tr/min. Pour les faibles vitesses d'agitation, on utilise par exemple un homogénéiseur tel qu'un défloculeur ; alors que pour les vitesses d'agitation de plus de 8000 tr/min on utilise un dispositif type Ultra-turrax. Le procédé de l'invention comprend l'utilisation d'un COPA avec des fins de chaîne majoritairement acides (c'est-à-dire à plus de 50% en poids acides) dans un milieu (de préférence aqueux) comprenant un composé basique qui réagit avec les fins de chaîne acides pour donner des carboxylate, ceux-ci jouant le rôle de tensioactif. De préférence, le ratio des groupes carboxyles terminaux sur les groupes amino terminaux du COPA utilisé dans la présente invention est compris dans la gamme de 60/40 à 95/5.
Par composé basique, on entend un hydroxyde de métal alcalin ou un composé amino. A titre d'exemples, on peut citer l'hydroxyde de sodium ou l'hydroxyde de potassium.
De préférence, la composition selon l'invention comprend de 0,2 à 3,0 moles de composé basique par mole de groupes carboxyle terminaux du COPA.
De préférence, le COPA est dispersé dans ledit milieu (de préférence aqueux) à une température supérieure ou égale à la température de fusion Tf du COPA, de préférence à une température comprise dans la gamme de 70 à 200°C, de préférence de 90 à 190°C.
Les dispersions aqueuses de l'exemple 1 ci-après sont conformes à ces paramètres préférés.

La présente invention a encore pour objet l'utilisation d'un copolyamide (COPA) pour la fabrication d'un produit cosmétique, pharmaceutique ou de parfumerie, ledit COPA étant incorporé sous la forme d'une composition conforme à l'invention.

La présente invention a notamment pour objet une composition selon l'invention telle que définie précédemment, ladite composition étant un produit coloré, non coloré et/ou transparent choisi parmi les produits suivants :
- produits de maquillage pour le visage et le corps humain, tels que fond de teint, crème teintée, poudre libre ou compacte, fard à paupières, mascara, eye liner, rouge à lèvre, vernis à ongles ;
- produits de soins pour le visage et le corps humain, tels que crème, lait, lotion, masque, produit de gommage, produits nettoyants et/ou démaquillants, déodorants, antitranspirants, antiperspirants, produits de rasage, produits d'épilation ;

- produits capillaires, tels que shampooings, produits pour la mise en forme des cheveux, produits de maintien de la coiffure, produits antipelliculaires, produits antichute, produits contre la sécheresse des cheveux, teintures capillaires, produits de décoloration ;
- produits de parfumerie, tels que parfum, lait, crème, poudre libre ou compacte parfumée.

### Exemples

Les exemples ci-dessous illustrent la présente invention sans en limiter la portée. Sauf indication contraire, tous les pourcentages sont en poids.

### Exemple 1

### COPA utilisés :

COPA 1 : copolyamide Pip.10/12 de ratio (pourcentage massique : 72/28), « Pip » étant la pipérazine.
COPA 2 [hors invention]: copolyamide 6/6.6/12 de ratio (pourcentage massique: 35/20/45).

On fabrique une dispersion aqueuse de COPA1 puis de COPA2 selon le même procédé suivant :
- ajouter 15% en poids de COPA à de l'eau (84%) mélangée à 1% de NaOH, sous agitation (300 tr/min) et à une température maintenue au dessus du point de fusion du COPA, ici maintenue à 150°C, et homogénéiser jusqu'à obtenir une dispersion aqueuse de particules de COPA de D50 comprise dans la gamme de 0,5 à 5 µm ; puis
- laisser refroidir jusqu'à température ambiante (25°C).

Une dispersion aqueuse de COPA 1 et une dispersion aqueuse de COPA 2 (compositions selon l'invention) sont respectivement obtenues à l'exemple 1 cosmétique comme dans les exemples suivants :

### Exemple 2

**Formulation de crème :**

| | **Crème** | % |
|---|---|---|
| phase A (huileuse) | acide stéarique | 12,5 |
| | alcool cétylique | 0,5 |
| | | |
| phase B (acqueuse) | triéthylamine | 2 |
| | glycérine | 10 |
| | Dispersion COPA1 de l'Exemple 1 | 7 |
| | eau | qsp 100 |
| | conservateur (Germall plus) | 0,1 |

### Mode opératoire :

- Chauffer séparément A et B à une température d'environ 90°C ;
- Verser B dans A, sous agitation (au défloculeur, type homogénéiseur Silverson®) pendant au moins 5 minutes et jusqu'à obtenir une émulsion homogène ;
- Continuer l'agitation lente en diminuant la température progressivement jusqu'à 30°C et ajouter le conservateur.

On obtient une crème blanche protectrice, d'aspect « moelleux » lors de la prise et de l'application.

### Example 3 [hors invention]

**Formulation de lait démaquillant :**

| | **Lait démaquillant** | % |
|---|---|---|
| phase A (huileuse) | acide stéarique | 3,5 |
| | huile de paraffine | 9 |
| | alcool cétylique | 0,9 |
| | Monostéarate de PEG (Tefose 1500) | 0,9 |
| | | |
| phase B (acqueuse) | triéthylamine | 1,8 |
| | eau | qsp 100 |
| | Dispersion COPA 2 de l'Exemple 1 | 5 |
| | eau | qsp 100 |
| | conservateur (Phenonip) | 0,2 |

### Mode opératoire :

- Chauffer séparément A et B à 80°C
- Verser lentement B dans A, sous agitation (au défloculeur) pendant au moins 5 minutes et jusqu'à obtenir une émulsion homogène ;
- Continuer l'agitation lente en diminuant la température progressivement jusqu'à 30°C et ajouter le conservateur.

On obtient un lait fluide démaquillant, qui appliqué sur la peau, absorbe efficacement les impuretés (maquillage, excès de sébum) de la peau.

### Exemple 4

**Formulation de mascara H/E :**

| | **Mascara H/E** | % |
|---|---|---|
| phase A (huileuse) | cire d'abeille | 5 |
| | ozokerite | 7 |
| | cire de Carnauba | 3 |
| | acide stéarique | 5 |
| | glycéryl stéarique | 5 |
| | Sepicid | 0,5 |
| | | |
| phase B (acqueuse) | eau | qsp 100 |
| | triéthylamine | 1,5 |
| | propylène glycol | 5 |
| | oxyde de fer noir | 10 |
| | Dispersion COPA 1 de l'Exemple 1 | 7,5 |
| | | |
| actif | eau | 1 |
| | D-panthénol | 0,5 |

### Mode opératoire :

- Chauffer la phase huileuse à 90°C ;
- Disperser le pigment (oxyde de fer noir) à froid à l'ultraturax dans l'eau avec le propylène glycol et la triéthylamine ;
- Chauffer la phase aqueuse ainsi obtenue à 90°C et y ajouter la dispersion aqueuse de COPA 1 de l'Exemple 1;
- Verser la phase B aqueuse dans la phase A huileuse sous agitation rapide (200 tr/min) au moyen d'un défloculeur pendant 5 minutes ;
- Refrodir l'émulsion obtenue sous agitation modérée jusqu'à une température de 25-30°C, puis ajouter l'actif.

On obtient un mascara recourbant les cils de manière « flexible ». La composition selon l'invention procure un effet filmogène, gainant et recourbant les cils tout en souplesse.

## Revendications

1. Composition comprenant :
- de 0,1 à 30% en poids de copolyamide (COPA) et
- de 70 à 99,9% en poids d'un milieu acceptable en cosmétique, en parfumerie et/ou en pharmacie,
- le dit milieu comprenant une phase aqueuse comprenant au moins 50% d'eau, et
- ledit copolyamide étant le PA PIP.10/12 de ratio massique 72/28 ;
- ledit COPA comprenant des fins de chaînes majoritairement acides et ledit milieu comprenant un composé basique, de préférence choisi parmi un hydroxyde de métal alcalin ou un composé amino.

2. Composition selon la revendication 1, dans laquelle ledit milieu comprend au moins un composant choisi parmi l'eau, les alcools, les solutions alcooliques, les composés lipidiques, les composés glucidiques, les hydrocarbures, les polymères synthétiques, polymères naturels, et/ou les extraits végétaux.

3. Composition selon la revendication 1 ou 2, ladite composition ayant au moins l'une des formes suivantes : dispersion, solution, émulsion, microémulsion, nanoémulsion, émulsion sèche, suspension, aérosol, gel, gel compact, gomme, gomme plastique, pâte, mousse, crème, poudre, poudre libre, poudre compacte, poudre expansée, beurre, film, film élastique, et leurs mélanges.

4. Composition selon l'une quelconque des revendications 1 à 3, dans laquelle ledit milieu comprend une phase huileuse comprenant au moins 50% de solvant organique choisi parmi les esters gras, les alcools gras, les acides gras et leurs mélanges.

5. Composition selon l'une quelconque des revendications 1 à 4 dans laquelle ledit milieu comprend de 0,1 à 30% en poids de tensioactif sur le poids total de COPA, de préférence choisi parmi les tensioactifs anioniques et les tensioactifs non ioniques, de préférence choisi parmi les sels de sulfate d'éther d'alkyle et de polyoxyalkylène, les sels de dialkylsulfosuccinate, les sels d'acide gras et les copolyamides d'oxyde d'éthylène/oxyde de propylène, et leurs mélanges.

6. Composition selon l'une quelconque des revendications 1 à 5, comprenant de 0,2 à 3,0 moles de composé basique par mole de groupes carboxyle terminaux du COPA.

7. -Composition selon l'une quelconque des revendications 1 à 6, comprenant en outre un épaississant.

8. -Composition selon l'une quelconque des revendications 1 à 7, **caractérisée en ce qu'**elle forme une dispersion de COPA dissous dans une phase huileuse dispersée dans une phase aqueuse en présence d'un tensioactif.

9. -Composition selon l'une quelconque des revendications 1 à 8, **caractérisée en ce qu'**elle forme une dispersion de COPA dissous dans une phase acqueuse dispersée dans une phase huileuse en présence d'un tensioactif.

10. -Procédé d'incorporation d'un copolyamide tel que défini à l'une quelconque des revendications 1 à 9 dans un milieu cosmétique, de parfumerie et/ou pharmaceutique, le dit milieu comprenant une phase aqueuse comprenant au moins 50% d'eau, et ledit milieu comprenant un composé basique, de préférence choisi parmi un hydroxyde de métal alcalin ou un composé amino, le ratio en poids COPA/milieu étant compris dans la gamme de 40/60 à 1/99, les bornes de la gamme étant incluses, ledit procédé comprenant au moins une étape choisie parmi : mélanger, disperser, homogénéiser, homogénéiser par haute pression, mettre en solution, diluer, dissoudre, gélifier, épaissir, émulsionner, affiner, empâter, traiter thermiquement, sécher, lyophiliser, cuire, extruder, broyer, granuler, atomiser, filtrer, et les mélanges successifs ou simultanés de plusieurs de ces étapes.

11. Procédé selon la revendication 10, comprenant les étapes de :
- ajout de copolyamide (COPA) dans un milieu acceptable en cosmétique, en parfumerie, et/ou en pharmacie, le ratio en poids COPA/milieu étant compris dans la gamme de 40/60 à 1/99, les bornes de la gamme étant incluses; et
- chauffage dudit mélange à une température comprise dans la gamme de 40 à 190°C, pendant une durée comprise dans la gamme de 5 à 120 minutes;
les étapes d'ajout et de chauffage étant soit simultanées, soit successives prises dans cet ordre ou dans l'ordre inverse.

12. Procédé selon la revendication 10 ou 11, dans lequel l'étape d'ajout comprend l'ajout de 0,1 à 30% de copolyamide (COPA) dans 70 à 99,9% d'un milieu acceptable en cosmétique et/ou en parfumerie, le mélange obtenu représentant 100%.

13. Procédé selon l'une quelconque des revendications 10 à 12, dans lequel le COPA utilisé est sous forme de poudre de D50 compris dans la gamme de 1 à 150 µm.

14. Procédé selon l'une quelconque des revendications 10 à 13 dans lequel le milieu acceptable en cosmétique, en parfumerie et/ou en pharmacie comprend une phase aqueuse comprenant au moins 50% d'eau sur le poids de phase aqueuse et une phase huileuse comprenant au moins 50% de solvant organique sur le poids de phase huileuse, ledit solvant organique étant choisi parmi les esters gras, les alcools gras, les acides gras et leurs mélanges.

15. Procédé selon la revendication 14, comprenant les étapes suivantes :
- dissoudre le COPA dans la phase huileuse ;
- émulsionner la solution de COPA obtenue dans la phase aqueuse contenant au moins un tensioactif choisi parmi les tensioactifs anioniques et les tensioactifs non ioniques.

16. Procédé selon l'une quelconque des revendications 10 à 15, comprenant une étape consistant à disperser du COPA de température de fusion Tf dans la phase aqueuse contenant au moins un composé basique et/ou un tensioactif choisi parmi les tensioactifs anioniques et les tensioactifs non ioniques, à une température supérieure ou égale à Tf, et sous agitation à taux de cisaillement suffisant pour que le D50 des gouttes de COPA émulsionnées soit dans la gamme de 0,1 à 50 µm.

17. -Utilisation d'un copolyamide (COPA) pour la fabrication d'un produit cosmétique, pharmaceutique ou de parfumerie, ledit COPA étant incorporé sous la forme d'une composition conforme à l'une des revendications 1 à 9.

18. -Composition selon l'une quelconque des revendications 1 à 9, ladite composition étant un produit coloré, non coloré et / ou transparent choisi parmi les produits suivants :
- produits de maquillage pour le visage et le corps humain, tels que fond de teint, crème teintée, poudre libre ou compacte, fard à paupières, mascara, eye liner, rouge à lèvre, vernis à ongles ;
- produits de soins pour le visage et le corps humain, tels que crème, lait, lotion, masque, produit de gommage, produits nettoyants et/ou démaquillants, déodorants, antitranspirants, antiperspirants, produits de rasage, produits d'épilation ;
- produits capillaires, tels que shampooings, produits pour la mise en forme des cheveux, produits de maintien de la coiffure, produits antipelliculaires, produits antichute, produits contre la sécheresse des cheveux, teintures capillaires, produits de décoloration ;
- produits de parfumerie, tels que parfum, lait, crème, poudre libre ou compacte parfumée.

## Patentansprüche

1. Zusammensetzung, umfassend:
- 0,1 bis 30 Gew.-% Copolyamid (COPA) und
- 70 bis 99,9 Gew.-% eines in Kosmetik, Parfümerie und/oder Pharmazie unbedenklichen Mediums,
- wobei das Medium eine wässrige Phase umfasst, die mindestens 50% Wasser umfasst, und
- wobei es sich bei dem Copolyamid um PA PIP.10/12 mit einem Massenverhältnis von 72/28 handelt;
- wobei das COPA überwiegend saure Kettenenden umfasst und das Medium eine basische Verbindung umfasst, vorzugsweise ausgewählt aus einem Alkalimetallhydroxid oder einer Aminoverbindung.

2. Zusammensetzung gemäß Anspruch 1, wobei das Medium mindestens eine aus Wasser, Alkoholen, alkoholischen Lösungen, Lipidverbindungen, Kohlenhydratverbindungen, Kohlenwasserstoffen, synthetischen Polymeren, natürlichen Polymeren und/oder Pflanzenextrakten ausgewählte Komponente umfasst.

3. Zusammensetzung gemäß Anspruch 1 oder 2, wobei die Zusammensetzung mindestens eine der folgenden Formen aufweist: Dispersion, Lösung, Emulsion, Mikroemulsion, Nanoemulsion, Trockenemulsion, Suspension, Aerosol, Gel, Kompaktgel, Gummi, Kunststoffgummi, Paste, Schaum, Creme, Puder, loser Puder, Kompaktpuder, geschäumter Puder, Butter, Film, elastischer Film und Mischungen davon.

4. Zusammensetzung gemäß einem der Ansprüche 1 bis 3, wobei das Medium eine Ölphase umfasst, die mindestens 50 % organisches Lösungsmittel umfasst, ausgewählt aus Fettestern, Fettalkoholen, Fettsäuren und Mischungen davon.

5. Zusammensetzung gemäß einem der Ansprüche 1 bis 4, wobei das Medium 0,1 bis 30 Gew.-% Tensid bezogen auf das Gesamtgewicht des COPA umfasst, vorzugsweise ausgewählt aus anionischen Tensiden und nichtionischen Tensiden, vorzugsweise ausgewählt aus Salzen von Alkylethersulfat und von Polyoxyalkylen, Dialkylsulfosuccinatsalzen, Fettsäuresalzen und Ethylenoxid/Propylenoxid-Copolyamiden sowie Mischungen davon.

6. Zusammensetzung gemäß einem der Ansprüche 1 bis 5, umfassend 0,2 bis 3,0 Mol basische Verbindung pro Mol COPA-terminale Carboxylgruppen.

7. Zusammensetzung gemäß einem der Ansprüche 1 bis 6, ferner umfassend ein Verdickungsmittel.

8. Zusammensetzung gemäß einem der Ansprüche 1 bis 7, **dadurch gekennzeichnet, dass** sie eine COPA-Dispersion bildet, die in einer Ölphase gelöst ist, die in einer wässrigen Phase in Gegenwart eines Tensids dispergiert ist.

9. Zusammensetzung gemäß einem der Ansprüche 1 bis 8, **dadurch gekennzeichnet, dass** sie eine COPA-Dispersion bildet, die in einer wässrigen Phase gelöst ist, die in einer Ölphase in Gegenwart eines Tensids dispergiert ist.

10. Verfahren zum Beimengen eines Copolyamids gemäß einem der Ansprüche 1 bis 9 in ein Kosmetik-, Parfümerie- und/oder Pharmazie-Medium, wobei das Medium eine wässrige Phase umfasst, die mindestens 50% Wasser umfasst, und das Medium eine basische Verbindung umfasst, vorzugsweise ausgewählt aus einem Alkalimetallhydroxid oder einer Aminoverbindung, wobei das Gewichtsverhältnis COPA/Medium im Bereich von 40/60 bis 1/99 liegt, die Grenzen des Bereichs mit eingeschlossen, und das Verfahren mindestens einen Schritt umfasst, der ausgewählt ist aus: Mischen, Dispergieren, Homogenisieren, Homogenisieren durch Hochdruck, Lösen, Verdünnen, Auflösen, Gelieren, Eindicken, Emulgieren, Verfeinern, Pastinieren, Wärmebehandeln, Trocknen, Gefriertrocknen, Kochen, Extrudieren, Mahlen, Granulieren, Zerstäuben, Filtern und aufeinanderfolgenden oder gleichzeitigen Kombinationen mehrerer dieser Schritte.

11. Verfahren gemäß Anspruch 10, umfassend die Schritte:
- Zugeben von Copolyamid (COPA) in ein in Kosmetik, Parfümerie und/oder Pharmazie unbedenkliches Medium, wobei das Gewichtsverhältnis von COPA/Medium im Bereich von 40/60 bis 1/99 liegt, die Grenzen des Bereichs mit eingeschlossen; und
- Erhitzen der Mischung auf eine Temperatur im Bereich von 40 bis 190°C für eine Zeitdauer im Bereich von 5 bis 120 Minuten;
wobei die Schritte des Zugebens und Erhitzens entweder gleichzeitig oder nacheinander in dieser Reihenfolge oder in umgekehrter Reihenfolge durchgeführt werden.

12. Verfahren gemäß Anspruch 10 oder 11, wobei der Schritt des Zugebens die Zugabe von 0,1 bis 30 % Copolyamid (COPA) in 70 bis 99,9 % eines in Kosmetik und/oder Parfümerie unbedenklichen Mediums umfasst, wobei die resultierende Mischung 100 % beträgt.

13. Verfahren gemäß einem der Ansprüche 10 bis 12, wobei das verwendete COPA in Form von D50-Pulver im Bereich von 1 bis 150 µm vorliegt.

14. Verfahren gemäß einem der Ansprüche 10 bis 13, wobei das in Kosmetik, Parfümerie und/oder Pharmazie unbedenkliche Medium eine wässrige Phase umfasst, die mindestens 50 % Wasser bezogen auf das Gewicht der wässrigen Phase umfasst, und eine Ölphase, die mindestens 50 % organisches Lösungsmittel bezogen auf das Gewicht der Ölphase umfasst, wobei das organische Lösungsmittel aus Fettestern, Fettalkoholen, Fettsäuren und Mischungen davon ausgewählt ist.

15. Verfahren gemäß Anspruch 14, umfassend die folgenden Schritte:
- Auflösen des COPA in der Ölphase;
- Emulgieren der in der wässrigen Phase erhaltenen COPA-Lösung, die mindestens ein Tensid enthält, ausgewählt aus anionischen Tensiden und nichtionischen Tensiden.

16. Verfahren gemäß einem der Ansprüche 10 bis 15, umfassend einen Schritt des Dispergierens von COPA mit einer Schmelztemperatur Tf in der wässrigen Phase, enthaltend mindestens eine basische Verbindung und/oder ein Tensid, ausgewählt aus anionischen Tensiden und nichtionischen Tensiden, bei einer Temperatur größer oder gleich Tf und unter Rühren mit einer Schergeschwindigkeit, die ausreicht, damit das D50 der emulgierten COPA-Tropfen in dem Bereich zwischen 0,1 und 50 µm liegt.

17. Verwendung eines Copolyamids (COPA) zur Herstellung eines kosmetischen, pharmazeutischen oder Parfümerie-Produkts, wobei das COPA in Form einer Zusammensetzung nach einem der Ansprüche 1 bis 9 enthalten ist.

18. Zusammensetzung gemäß einem der Ansprüche 1 bis 9, wobei die Zusammensetzung ein gefärbtes, ungefärbtes und/oder transparentes Produkt ist, das unter den folgenden Produkten ausgewählt ist:
- Make-up-Produkte für Gesicht und Körper eines Menschen, wie Foundation, getönte Creme, loser oder kompakter Puder, Lidschatten, Mascara, Eyeliner, Lippenstift und Nagellack;
- Gesichts- und Körperpflegeprodukte für Menschen, wie Creme, Milch, Lotion, Masken, Peelings, Reinigungs- und/oder Make-up-Entfernungsprodukte, Deodorants, Antitranspirantien, Antiperspirantien, Rasiermittel, Haarentfernungsprodukte;
- Haarprodukte, wie Shampoos, Haarformungsprodukte, Haarstylingprodukte, Anti-Schuppen-Produkte, Produkte gegen Haarausfall, Produkte gegen trockenes Haar, Haarfärbemittel, Bleichmittel;
- Parfümerieprodukte wie Parfüm, Milch, Creme, parfümierter loser oder kompakter Puder.

## Claims

1. Composition comprising:
- from 0.1 to 30% by weight of copolyamide (COPA), and
- from 70 to 99.9% by weight of a medium acceptable in cosmetics, in perfumery and/or in pharmaceuticals,
- said medium comprising an aqueous phase comprising at least 50% water, and
- said copolyamide being PA PIP. 10/12 with a ratio by weight of 72/28;
- said COPA comprising predominantly acid chain ends and said medium comprising a basic compound, preferably chosen from an alkali metal hydroxide or an amino compound.

2. Composition according to Claim 1, in which said medium comprises at least one compound chosen from water, alcohols, alcoholic solutions, lipid compounds, glucide compounds, hydrocarbons, synthetic polymers, natural polymers and/or plant extracts.

3. Composition according to Claim 1 or Claim 2, said composition having at least one of the following forms: dispersion, solution, emulsion, microemulsion, nanoemulsion, dry emulsion, suspension, aerosol, gel, compact gel, gum, plastic gum, paste, foam, cream, powder, loose powder, compact powder, expanded powder, butter, film, elastic film and their mixtures.

4. Composition according to any one of Claims 1 to 3, in which said medium comprises an oily phase comprising at least 50% of organic solvent chosen from fatty esters, fatty alcohols, fatty acids and their mixtures.

5. Composition according to any one of Claims 1 to 4, in which said medium comprises from 0.1 to 30% by weight of surfactant with regard to the total weight of COPA, preferably chosen from anionic surfactants and nonionic surfactants, preferably chosen from salts of alkyl ether sulfate and of polyoxyalkylene, dialkyl sulfosuccinate salts, fatty acid salts and copolyamides of ethylene oxide/propylene oxide, and their mixtures.

6. Composition according to any one of Claims 1 to 5, comprising from 0.2 to 3.0 mol of basic compound per mole of end carboxyl groups of the COPA.

7. Composition according to any one of Claims 1 to 6, additionally comprising a thickener.

8. Composition according to any one of Claims 1 to 7, **characterized in that** it forms a dispersion of COPA dissolved in an oily phase dispersed in an aqueous phase in the presence of a surfactant.

9. Composition according to any one of Claims 1 to 8, **characterized in that** it forms a dispersion of COPA dissolved in an aqueous phase dispersed in an oily phase in the presence of a surfactant.

10. Method for the incorporation of a copolyamide as defined in any one of Claims 1 to 9 in a cosmetic, perfumery and/or pharmaceutical medium, said medium comprising an aqueous phase comprising at least 50% water, and said medium comprising a basic compound, preferably chosen from an alkali metal hydroxide or an amino compound, the COPA/medium ratio by weight being within the range from 40/60 to 1/99, the limits of the range being included, said method comprising at least one stage chosen from: mixing, dispersing, homogenizing, high pressure homogenizing, dissolving, diluting, gelling, thickening, emulsifying, refining, forming a paste, heat treating, drying, lyophilizing, baking, extruding, grinding, granulating, atomizing, filtering and the successive or simultaneous mixtures of several of these stages.

11. Method according to Claim 10, comprising the stages of:
- addition of copolyamide (COPA) to a medium acceptable in cosmetics, in perfumery and/or in pharmaceuticals, the COPA/medium ratio by weight being within the range from 40/60 to 1/99, the limits of the range being included; and
- heating said mixture at a temperature within the range from 40 to 190°C, for a period of time within the range from 5 to 120 minutes;
the addition and heating stages being either simultaneous or successive, taken in this order or in the reverse order.

12. Method according to Claim 10 or Claim 11, in which the addition stage comprises the addition of from 0.1 to 30% of copolyamide (COPA) to from 70 to 99.9% of a medium acceptable in cosmetics and/or in perfumery, the mixture obtained representing 100%.

13. Method according to any one of Claims 10 to 12, in which the COPA used is in the form of a powder with a D50 within the range from 1 to 150 µm.

14. Method according to any one of Claims 10 to 13, in which the medium acceptable in cosmetics, in perfumery and/or in pharmaceuticals comprises an aqueous phase comprising at least 50% of water with regard to the weight of aqueous phase and an oily phase comprising at least 50% of organic solvent with regard to the weight of oily phase, said organic solvent being chosen from fatty esters, fatty alcohols, fatty acids and their mixtures.

15. Method according to Claim 14, comprising the following stages:
- dissolving the COPA in the oily phase;
- emulsifying the COPA solution obtained in the aqueous phase comprising at least one surfactant chosen from anionic surfactants and nonionic surfactants.

16. Method according to any one of Claims 10 to 15, comprising a stage consisting in dispersing COPA with a melting point M.p. in the aqueous phase comprising at least one basic compound and/or one surfactant chosen from anionic surfactants and nonionic surfactants, at a temperature of greater than or equal to M.p., and with stirring at a shear rate sufficient for the D50 of the emulsified COPA drops to be within the range from 0.1 to 50 µm.

17. Use of a copolyamide (COPA) in the manufacture of a cosmetic, pharmaceutical or perfumery product, said COPA being incorporated in the form of a composition in accordance with one of Claims 1 to 9.

18. Composition according to any one of Claims 1 to 9, said composition being a colored, colorless and/or transparent product chosen from the following products:
- makeup products for the human face and body, such as foundation, tinted cream, loose or compact powder, eyeshadow, mascara, eyeliner, lipstick or nail varnish;
- care products for the human face and body, such as cream, milk, lotion, mask, scrubber, cleansing and/or makeup-removing products, deodorants, antiperspirants, shaving products or hair-removing products;
- hair products such as shampoos, products for the shaping of the hair, products for retaining the hairstyle, antidandruff products, products for combating hair loss, products for combating dryness of the hair, hair dyes or bleaching products;
- perfumery products, such as fragrance, milk, cream, or loose or compact scented powder.
